**Europäisches Patentamt**

**European Patent Office**

**Office européen des brevets**

(11) Veröffentlichungsnummer: **0 064 633**
**B1**

(12) **EUROPÄISCHE PATENTSCHRIFT**

(45) Veröffentlichungstag der Patentschrift:
**12.09.84**

(21) Anmeldenummer: **82103380.0**

(22) Anmeldetag: **21.04.82**

(51) Int. Cl.³: **C 07 C 55/02, C 07 C 51/31**

(54) Verfahren zur Herstellung von 3,3-Dimethylglutarsäure.

(30) Priorität: **12.05.81 CH 3062/81**

(43) Veröffentlichungstag der Anmeldung:
**17.11.82 Patentblatt 82/46**

(45) Bekanntmachung des Hinweises auf die Patenterteilung:
**12.09.84 Patentblatt 84/37**

(84) Benannte Vertragsstaaten:
**AT BE CH DE FR GB IT LI LU NL SE**

(56) Entgegenhaltungen:
**FR - A - 1 066 259**

(73) Patentinhaber: **LONZA AG, Gampel/Wallis (CH)**

(72) Erfinder: **Lehky, Pavel, Dr., Dammweg 13, Naters
(Kanton Wallis) (CH)**
Erfinder: **Franzen, Volker, Prof. Dr., C.F.
Meyer-Strasse 32, Basel (CH)**

(74) Vertreter: **Weinhold, Peter, Dr. et al, Patentanwälte Dr. V.
Schmied-Kowarzik Dipl.-Ing. G. Dannenberg Dr. P.
Weinhold Dr. D. Gudel Dipl.-Ing. S. Schubert Dr. P. Barz
Siegfriedstrasse 8, D-8000 München 40 (DE)**

## Beschreibung

Die Erfindung betrifft ein Verfahren zur Herstellung von 3,3-Dimethylglutarsäure aus Isophoron.

3,3-Dimethylglutarsäure und ihre Ester stellen Zwischenprodukte bei der Herstellung von Pestiziden (DE-OS 2813341) dar. Ferner sind sie als Zusatz für Schmieröl geeignet (US-PS 2971915).

Es ist bekannt, Isophoron mit $H_2O_2$ in alkalischem Medium zu behandeln (R.D. Temple, J. Org. Chem. 35, 1275). Dabei wird eine Ausbeute von 92% 3,3-Dimethyl-5-oxo-hexansäure erhalten. Andererseits sind für die Herstellung von 3,3-Dimethylglutarsäure aus der Literatur verschiedene Methoden bekannt. So wird nach W.T. Schmith und G.L. McLeod, Org. Synthesis 31, 40 (1951) durch Oxidation von Dimedon (5,5-Dimethyl-1,3-cyclohexandion) mit Natriumhypochlorit 3,3-Dimethylglutarsäure erhalten. F.B. Thole und J.F. Thorpe, J. Chem. Soc. 99, 422 (1911) erhalten Dimethylglutarsäure aus Aceton und Cyanacetanud. W.H. Perkin jun. und J.F. Thorpe, J. Chem. Soc. 75, 48 (1899) stellen Dimethylglutarsäure aus Dimethylacrylester und Cyanessigester her.

Alle diese bekannten Verfahren haben Nachteile, z.B. dass sie entweder von teuren Edukten ausgehen oder über mehrere Synthesestufen verlaufen oder niedere Ausbeuten bringen, viel Abfallsalz als Nebenprodukt produzieren oder stark verunreinigtes Endprodukt liefern.

Ziel der vorliegenden Erfindung ist es, 3,3-Dimethylglutarsäure, ausgehend von Isophoron, auf einfache Weise zu produzieren.

Erfindungsgemäss wird dies dadurch erreicht, dass man Isophoron mit Wasserstoffperoxid in Gegenwart starker Säuren bei Temperaturen von 10 bis 80°C behandelt.

Als starke Säuren kommen Protonensäuren (Brönstedsäuren) in Betracht, die einen pKa-Wert von 2,5 oder kleiner haben und die gegen Wasserstoffperoxid stabil sind, wie Schwefelsäure, Phosphorsäure, Salpetersäure, organische Sulfonsäuren, wie Benzolsulfonsäure, Trichloressigsäure, Trifluoressigsäure u.a. Bevorzugt wird die Verwendung von Schwefelsäure.

Nach speziellen Ausführungsformen der Erfindung wird wie folgt vorgegangen: Für die Durchführung der Reaktion wird in einem ersten Schritt ein Gemisch von Wasser, Wasserstoffperoxid und einer starken Säure hergestellt, indem man eine Lösung von Wasserstoffperoxid, eine Säure oder eine wässerige Lösung einer Säure und gegebenenfalls Wasser vereinigt.

Wasserstoffperoxid wird als wässerige Lösung eingesetzt, wobei die Konzentration in einem weiten Bereich von 20% bis ca. 85% variieren kann. Bevorzugt werden technisch erhältliche Konzentrationen von 30% und 70% $H_2O_2$ in Wasser.

Die starke Säure kann in reiner Form oder als wässerige Lösung eingesetzt werden. Im Falle, wo als starke Säure Schwefelsäure verwendet wird, wird bevorzugt technische, konzentrierte Schwefelsäure eingesetzt.

Die Mengen und die Konzentrationen von Wasserstoffperoxidlösung, starker Säure und gegebenenfalls Wasser werden so gewählt, dass auf 1 Mol Isophoron 4-8 Mol Wasserstoffperoxid, 4-8 Mol starke Säure und 20-40 Mol Wasser vorliegen.

Das bevorzugte Verhältnis liegt bei 5-6 Mol Wasserstoffperoxid, 5-6 Mol Schwefelsäure und 30 Mol Wasser auf 1 Mol Isophoron.

Die Reaktionstemperaturen werden vorteilhaft zwischen 20 und 50°C gewählt.

Nach dem erfindungsgemässen Verfahren wird 3,3-Dimethylglutarsäure in einer Reinheit von über 80% und in einer Ausbeute von bis über 75% erzielt.

*Beispiel 1*

In einem Dreihalskolben wurden 166,3 g 95%ige $H_2SO_4$ vorgelegt. Dazu wurden 200 g 30%iges Wasserstoffperoxid unter Kühlung auf ~10°C zugetropft. Während einer Stunde wurden 40 g Isophoron (99%ig) so zudosiert, dass die Temperatur auf 25°C konstant blieb. Nach der Zugabe wurde die Temperatur progressiv auf 58°C erhöht; bei dieser Temperatur wurde die Reaktionsmischung noch 16 Stunden gerührt. Die Mischung wurde auf −10°C gekühlt und der Kristallbrei abgenutscht. Die Kristalle enthielten 23,32 g Dimethylglutarsäure.

Die Reinheit des Produktes betrug 82%.

Die Mutterlauge wurde (nach Peroxidvernichtung durch Durchblasen von $SO_2$) nach Zugabe von konz. NaOH bis pH 1,5 dreimal mit 120 ml Äther extrahiert.

Der Extrakt nach Eindampfen von Äther enthielt noch 7,4 g als 100% rein gerechnete Dimethylglutarsäure.

Die totale Ausbeute, bezogen auf die eingesetzte Menge Isophoron, betrug 57,8%.

*Beispiel 2*

In einem Dreihalskolben wurden 280 g 56%ige $H_2SO_4$ vorgelegt. Dazu wurden unter Kühlung auf ~10°C 85 g 70%iges Wasserstoffperoxid zugetropft. Während einer Stunde wurden 40 g Isophoron so zudosiert, dass die Temperatur zwischen 25 und 30°C blieb. Nach der Zugabe wurde die Temperatur progressiv auf 58°C erhöht; bei dieser Temperatur wurde die Reaktionsmischung noch 5 Stunden gerührt. Die Mischung wurde langsam auf −10°C gekühlt und der Kristallbrei abgenutscht. Die Kristalle enthielten 29,7 g Dimethylglutarsäure mit einem Gehalt von 82,3% GC, mit internem Standard, entsprechend 24,44 g gebildeter Dimethylglutarsäure. Die Mutterlauge wurde (nach Peroxidvernichtung durch Durchblasen von $SO_2$) nach Zugabe von konz. NaOH bis pH 1,5 dreimal mit 120 ml Äther extrahiert.

Nach dem Eindampfen lassen sich im Extrakt mit Gaschromatographie (GC) weitere 7,50 g Dimethylglutarsäure nachweisen. Zusammen wurden 31,94 g Dimethylglutarsäure gebildet, entsprechend einer Ausbeute von 68,9%, bezogen auf eingesetztes Isophoron.

*Beispiel 3*

In einem Kolben wurden 83 g 85%ige Phosphorsäure vorgelegt. Dazu wurden 100 g 30%iges Wasserstoffperoxid zugetropft. Während einer Stunde wurden 20 g Isophoron so zudosiert, dass die Temperatur zwischen 25 bis 35°C blieb. Nach der beendeten Zugabe wurde die Reaktionsmischung bei Raumtemperatur weiter gerührt. Die Temperatur im Kolben stieg zuerst auf 50 bis 55°C, dann sank sie langsam während 3 bis 5 Stunden ab.

Die farblose bis leicht gelbe Lösung wurde von Peroxiden durch Behandlung mit $SO_2$ befreit und der pH-Wert mit konz. NaOH auf 1,5 eingestellt. Die gebildete Dimethylglutarsäure wurde durch Extraktion mit Äther isoliert. Laut der gaschromatographischen Analyse enthielt der Rückstand nach dem Eindampfen 8,87 g Dimethylglutarsäure, entsprechend einer Ausbeute von 38,3%, bezogen auf eingesetztes Isophoron.

## Patentansprüche

1. Verfahren zur Herstellung von 3,3-Dimethylglutarsäure aus Isophoron, dadurch gekennzeichnet, dass man Isophoron mit Wasserstoffperoxid in Gegenwart starker Säuren bei Temperaturen von 10 bis 80°C behandelt.

2. Verfahren nach Patentanspruch 1, dadurch gekennzeichnet, dass man als starke Säure Schwefelsäure verwendet.

3. Verfahren nach Patentanspruch 1, dadurch gekennzeichnet, dass man ein Molverhältnis von Isophoron zu Wasserstoffperoxid zu starker Säure von 1:(4-8):(4-8) verwendet.

4. Verfahren nach Patentansprüchen 1 bis 3, dadurch gekennzeichnet, dass man bei Temperaturen von 20 bis 50°C arbeitet.

## Claims

1. Process for the production of 3,3-dimethyl glutaric acid from isophorone, characterized by treating isophorone with hydrogen peroxide in the presence of strong acids at temperatures of from 10 to 80°C.

2. Process according to claim 1, characterized by using as strong acid sulfuric acid.

3. Process according to claim 1, characterized by using a molar ratio of isophorone to hydrogen peroxide to strong acid of 1:(4-8):(4-8).

4. Process according to any of claims 1 to 3, characterized by working at temperatures of from 20 to 50°C.

## Revendications

1. Procédé pour la préparation de l'acide 3,3-diméthylglutarique à partir de l'isophorone, caractérisé en ce qu'on traite de l'isophorone par du peroxyde d'hydrogène en présence d'acides forts à des températures de 10 à 80°C.

2. Procédé selon la revendication 1, caractérisé en ce qu'on utilise en tant qu'acide fort de l'acide sulfurique.

3. Procédé selon la revendication 1, caractérisé en ce qu'on utilise un rapport molaire de l'isophorone au peroxyde d'hydrogène à l'acide fort de 1:(4-8):(4-8).

4. Procédé selon les revendications 1 à 3, caractérisé en ce qu'on travaille à des températures de 20 à 50°C.